# EUROPEAN PATENT APPLICATION

(11) **EP 0 784 979 A2**
(43) Date of publication of application: **23.07.1997**
(21) Application number: 97300299.1
(22) Date of filing: 17.01.1997
(51) Int. Cl.: A61K 38/17

(54) **Obesity protein formulations**

(30) Priority: 07.02.1996 GB 9602408; 19.01.1996 US 10229
(71) Applicant: ELI LILLY AND COMPANY, Indianapolis, Indiana 46285 (US)
(72) Inventor: Beals, John Michael, Indianapolis, Indiana 46278-1853 (US); Dodd, Steven Witt, Indianapolis, Indiana 46254 (US); Pekar, Allen Howard, Indianapolis, Indiana 46220 (US)
(74) Representative: Hudson, Christopher Mark

(57) **Abstract**

The present invention discloses a soluble parenteral formulation, comprising obesity protein and a preservative selected from the group consisting of alkylparaben, chlorobutanol, or a mixture thereof.

## Description

The present invention is in the field of human medicine, particularly in the treatment of obesity and disorders associated with obesity. More specifically, the present invention relates to formulations of an obesity protein.

Obesity, and especially upper body obesity, is a common and very serious public health problem in the United States and throughout the world. According to recent statistics, more than 25% of the United States population and 27% of the Canadian population are overweight. Kuczmarski, Amer. J. of Clin. Nutr. 55: 495S - 502S (1992); Reeder et. al., Can. Med. Ass. J., 23: 226-233 (1992). Upper body obesity is the strongest risk factor known for type II diabetes mellitus, and is a strong risk factor for cardiovascular disease and cancer as well. Recent estimates for the medical cost of obesity are $150,000,000,000 world wide. The problem has become serious enough that the surgeon general has begun an initiative to combat the ever increasing adiposity rampant in American society.

Much of this obesity induced pathology can be attributed to the strong association with dyslipidemia, hypertension, and insulin resistance. Many studies have demonstrated that reduction in obesity by diet and exercise reduces these risk factors dramatically. Unfortunately, treatments are largely unsuccessful with a failure rate reaching 95%. This failure may be due to the fact that the condition is strongly associated with genetically inherited factors that contribute to increased appetite, preference for highly caloric foods, reduced physical activity, and increased lipogenic metabolism. This indicates that people inheriting these genetic traits are prone to becoming obese regardless of their efforts to combat the condition.

Therefore, a pharmacological agent that can correct this adiposity handicap and allow the physician to successfully treat obese patients in spite of their genetic inheritance is needed.

The *ob/ob* mouse is a model of obesity and diabetes that is known to carry an autosomal recessive trait linked to a mutation in the sixth chromosome. Recently, Yiying Zhang and co-workers published the positional cloning of the mouse gene linked with this condition. Yiying Zhang et al. Nature 372: 425-32 (1994). This report disclosed the murine and human protein expressed in adipose tissue. Likewise, Murakami et al., in Biochemical and Biophysical Research Communications 209(3):944-52 (1995) report the cloning and expression of the rat obese gene. The protein, which is encoded by the *ob* gene, has demonstrated an ability to effectively regulate adiposity in mice. Pelleymounter et al., Science 269: 540-543 (1995).

A parenteral formulation containing insoluble protein causes problems relating to inconsistency in the dose-response as well as unpredictability. The unpredictability is believed to be due to greater variability in the pharamacokinetics in suspension formulations. The insoluble formulations must first dissolve prior to adsorption. It is hypothesized that this step has significant variability in a subcutaneous depot. Furthermore, non-native association and aggregation under physiological conditions can lead to precipitation of the protein at the site of injection, which could lead to irritation or other immune response. For these reasons, a formulation of human obesity protein that developed insoluble protein particles would be unacceptable to patients seeking its benefits and to regulatory agencies.

Unfortunately, the naturally occurring obesity proteins demonstrate a propensity to aggregate making the preparation of a soluble, pharmaceutically acceptable parenteral formulation exceedingly difficult. The molecular interactions amongst the preservative, buffer, ionic strength, pH, temperature, and any additional excipients such as a surfactant, or sugar are highly unpredictable in view of the propensity for the obesity protein to aggregate and precipitate from the formulation.

The present invention provides conditions under which the obesity protein is soluble and commercially viable as a multi-use pharmaceutical product. Most unexpectedly, the physical stability of the formulation is greatly enhanced in the presence of methylparaben, propylparaben, butylparaben, chlorobutanol or a mixture thereof. That is, when formulated under the conditions described herein, the obesity protein remains soluble at much higher concentrations and at a pH range acceptable for a soluble, parenteral formulation. Accordingly, the present invention provides a soluble, parenteral formulations of an obesity protein.

This invention provides a soluble parenteral formulation, comprising an obesity protein and a preservative selected from the group consisting of alkylparaben, chlorobutanol, or a mixture thereof.

The invention further provides a process for preparing a soluble parenteral formulation, which comprises mixing an obesity protein and a preservative selected from the group consisting of alkylparaben, chlorobutanol, or a mixture thereof.

Additionally, the invention provides a method of treating obesity in a mammal in need thereof, which comprises administering to said mammal a soluble parenteral formulation of the present invention.

For purposes of the present invention, as disclosed and claimed herein, the following terms and abbreviations are defined as follows:

Alkylparaben -- refers to a C₁ to C₄ alkylparaben. Preferably, alkylparaben is methylparaben, ethylparaben, propylparaben, or butylparaben.

Base pair (bp) -- refers to DNA or RNA. The abbreviations A,C,G, and T correspond to the 5'-monophosphate forms of the nucleotides (deoxy)adenine, (deoxy)cytidine, (deoxy)guanine, and (deoxy)thymine, respectively, when they occur in DNA molecules. The abbreviations U,C,G, and T correspond to the 5'-monophosphate forms of the nucleosides uracil, cytidine, guanine, and thymine, respectively when they occur in RNA molecules. In double stranded DNA, base pair may refer to a partnership of A with T or C with G. In a DNA/RNA heteroduplex, base pair may refer to a partnership of T with A or C with G.

Obesity protein -- refers to the native mammalian protein produced from the native *ob* gene following transcription and deletions of introns, translation to a protein and processing to the mature protein with secretory signal peptide removed, e.g. from the N-terminal valine-proline to the C-terminal cysteine of the mature protein. The human obesity protein is published in Zhang *et al.* Nature 372: 425-32 (1994). The rat obesity protein is published in Murakami et al., Biochemical and Biophysical Research Comm. 209(3): 944-52 (1995). Native porcine and bovine Ob proteins are disclosed in a U.S. patent application by Hansen M. Hsiung and Dennis P. Smith filed May 19, 1995, Serial number 08/445,305 (EP0 743 321). Other mammalian Ob proteins are disclosed in U.S. patent application serial number 08/452,228, filed may 26, 1995 (EP0 744 408), U.S. provisional application, 60/003935, filed September 19, 1995 (BP-A 96306721.0). Obesity protein includes (those proteins having a leader sequence. A leader sequence is one or more amino acids on the N-terminus to aid in production or purification of the protein. A preferred leader sequence is Met-R1-wherein R1 is absent or any amino acid except Pro.

Plasmid -- an extrachromosomal self-replicating genetic element.

Reading frame -- the nucleotide sequence from which translation occurs "read" in triplets by the translational apparatus of tRNA, ribosomes and associated factors, each triplet corresponding to a particular amino acid. Because each triplet is distinct and of the same length, the coding sequence must be a multiple of three. A base pair insertion or deletion (termed a frameshift mutation) may result in two different proteins being coded for by the same DNA segment. To insure against this, the triplet codons corresponding to the desired polypeptide must be aligned in multiples of three from the initiation codon, i.e. the correct "reading frame" must be maintained. In the creation of fusion proteins containing a chelating peptide, the reading frame of the DNA sequence encoding the structural protein must be maintained in the DNA sequence encoding the chelating peptide.

Recombinant DNA Cloning Vector -- any autonomously replicating agent including, but not limited to, plasmids and phages, comprising a DNA molecule to which one or more additional DNA segments can or have been added.

Recombinant DNA Expression Vector -- any recombinant DNA cloning vector in which a promoter has been incorporated.

Replicon -- A DNA sequence that controls and allows for autonomous replication of a plasmid or other vector.

Transcription -- the process whereby information contained in a nucleotide sequence of DNA is transferred to a complementary RNA sequence.

Translation -- the process whereby the genetic information of messenger RNA is used to specify and direct the synthesis of a polypeptide chain.

Vector -- a replicon used for the transformation of cells in gene manipulation bearing polynucleotide sequences corresponding to appropriate protein molecules which, when combined with appropriate control sequences, confer specific properties on the host cell to be transformed. Plasmids, viruses, and bacteriophage are suitable vectors, since they are replicons in their own right. Artificial vectors are constructed by cutting and joining DNA molecules from different sources using restriction enzymes and ligases. Vectors include Recombinant DNA cloning vectors and Recombinant DNA expression vectors.

Treating -- as used herein, describes the management and care of a patient for the purpose of combating the disease, condition, or disorder and includes the administration of a protein of present invention to prevent the onset of the symptoms or complications, alleviating the symptoms or complications, or eliminating the disease, condition, or disorder. Treating as used herein includes the administration of the protein for cosmetic purposes. A cosmetic purpose seeks to control the weight of a mammal to improve bodily appearance.

Isotonicity agent -- isotonicity agent refers to an agent that is physiologically tolerated and embarks a suitable tonicity to the formulation to prevent the net flow of water across the cell membrane. Compounds, such as glycerin, are commonly used for such purposes at known concentrations. Other possible isotonicity agents include salts, e.g., NaCl, dextrose, mannitol, and lactose.

Physiologically tolerated buffer -- a physiologically tolerated buffer is known in the art. A physiologically tolerated buffer is preferably a phosphate buffer, like sodium phosphate. Other physiologically tolerated buffers include TRIS, sodium acetate, or sodium citrate. The selection and concentration of buffer is known in the art.

The nucleotide and amino acid abbreviations are accepted by the United States Patent and Trademark Office as set forth in 37 C.F.R. § 1.822 (b)(2) (1993). Unless otherwise indicated the amino acids are in the L configuration.

As noted above, the invention provides a soluble parenteral formulation, comprising human obesity protein and a preservative selected from the group consisting of an alkylparaben or chlorobutanol. In the presence of these preservatives, the obesity proteins remains in solution making a soluble, parenteral formulation possible.

A parenteral formulation must meet guidelines for preservative effectiveness to be a commercially viable product. Pharmaceutical preservatives known in the art as being acceptable in parenteral formulations include: phenol, m-cresol, benzyl alcohol, methylparaben, chlorobutanol, p-cresol, phenylmercuric nitrate, thimerosal and various mixtures thereof. See, e.g., WALLHAUSER, K.-H., DEVELOP. BIOL. STANDARD. 24, pp. 9-28 (Basel, S. Krager, 1974).

Most unexpectedly, a select number of preservatives have been identified which provide good formulation stability These select preservatives are an alkylparaben or chlorobutanol. Most preferrably, the preservative is methylparaben, propylparaben, or butylparaben. Most unexpectedly, the obesity protein does not aggregate in the presence of these preservatives at the conditions necessary to formulate and particularly conditions at 37°C.

The concentration of obesity protein in the formulation is about 1.0 mg/mL to about 100 mg/mL; preferably about 5.0 mg/mL to about 50.0 mg/mL; most preferably, about 10.0 mg/mL. The concentration of preservative required is the concentration necessary to maintain preservative effectiveness. The relative amounts of preservative necessary to maintain preservative effectiveness varies with the preservative used. Generally, the amount necessary can be found in WALLHAUSER, K.-H., DEVELOP. BIOL. STANDARD. 24, pp. 9-28 (Basel, S. Krager, 1974), herein incorporated by reference. The optimal concentration of the preservative depends on the preservative, its solubility, and the pH of the formulation.

An isotonicity agent, preferably glycerin, may be additionally added to the formulation. The concentration of the isotonicity agent is in the range known in the art for parenteral formulations, preferably about 1 to 20 mg/mL, more preferably about 8 to 16 mg/mL, and still more preferably about 16 mg/mL. The pH of the formulation may also be buffered with a physiologically tolerated buffer, preferably a phosphate buffer, like sodium phosphate. Other acceptable physiologically tolerated buffers include TRIS, sodium acetate, or sodium citrate. The selection and concentration of buffer is known in the art; however, the formulations of the present invention are preferably prepared the minimally accpetable concentration of buffer.

Other additives, such as a pharmaceutically acceptable solubilizers like Tween 20 (polyoxyethylene (20) sorbitan monolaurate), Tween 40 (polyoxyethylene (20) sorbitan monopalmitate), Tween 80 (polyoxyethylene (20) sorbitan monooleate), Pluronic F68 (polyoxyethylene polyoxypropylene block copolymers), BRIJ 35 (polyoxyethylene (23) lauryl ether), and PEG (polyethylene glycol) may optionally be added to the formulation to reduce aggregation. These additives are particularly useful if a pump or plastic container is used to administer the formulation. The presence of pharmaceutically acceptable surfactant mitigates the propensity for the protein to aggregate.

Also included as optional embodiments are agents known to be synergistic with the preservative to provide enhanced antimicrobial effect. Such agents are recognized in the art and include for example ethylene diaminetetraacetic acid (EDTA), 1,2-di(2-aminoethoxy)ethane-N,N,N'N'-tetraacetic acid (EGTA), citrate, and caprylic acid. The concentration of these agents varies with the desired preservation effect. A preferred agent is EDTA, particularly in conjunction with an alkylparaben at a concentration of about 0.025% to 0.4%. Notably, in preparations including EDTA or EGTA, the buffer concentration is reduced to minimize ionic strength.

The present formulations may optionally contain a physiologically tolerated solvent such as glycerol, propylene glycol, phenoxy ethanol, phenyl ethyl alcohol. Such solvents are generally added to enhance the solubility of the protein in the preservation effectiveness of the formulation.

The parenteral formulations of the present invention can be prepared using conventional dissolution and mixing procedures. To prepare a suitable formulation, for example, a measured amount of human obesity protein in water is combined with the desired preservative in water in quantities sufficient to provide the protein and preservative at the desired concentration. The formulation is generally sterile filtered prior to administration. Variations of this process would be recognized by one of ordinary skill in the art. For example, the order the components are added, the surfactant used, the temperature and pH at which the formulation is prepared may be optimized for the concentration and means of administration used.

The unexpected preservative effect on formulation stability was demonstrated by preparing formulations comprising methylparaben, propylparaben, butylparaben, chlorobutanol, cresol, phenol, benzyl alcohol, or a mixture thereof and comparing the amount of protein remaining in solution after 3 days at 4°C and 37°C. The data in Table 1 demonstrate that the stability and solubility of human obesity protein is enhanced in the presence of an alkylparaben or chlorobutanol. The formulations used to generate the data of Table 1 were prepared in a manner analogous to Examples 1 and 2.

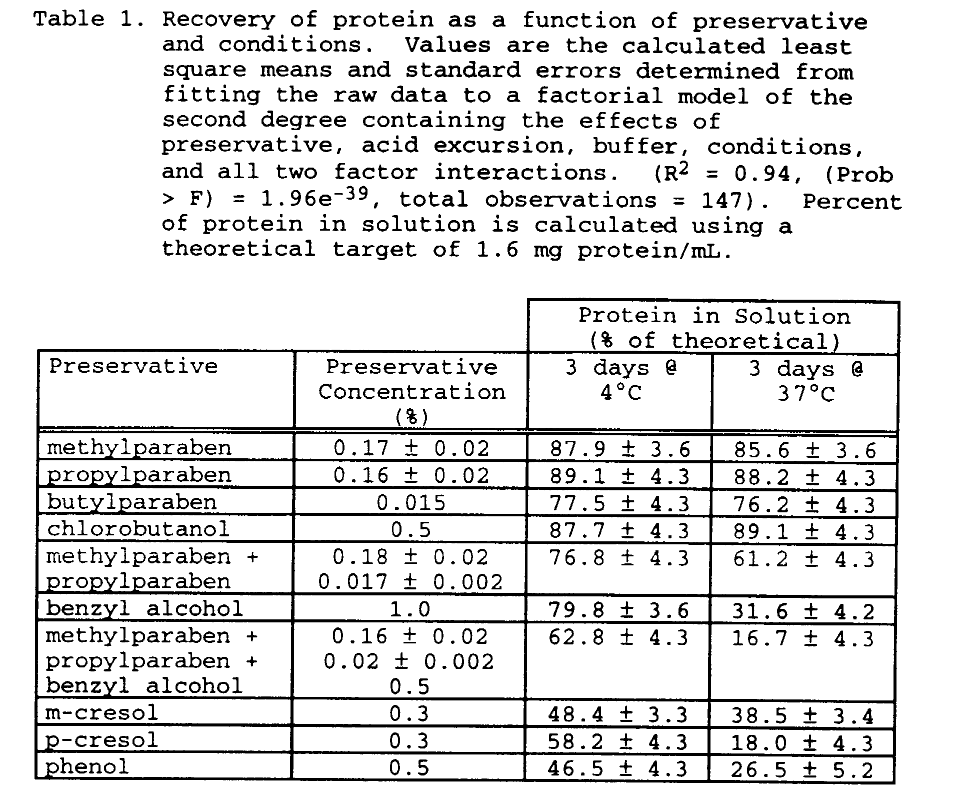

The stabilizing effect by the alkylparabens is most unexpected in view of the structural similarities to other preservatives. The data clearly show that the alkylparabens and chlorobutanol are superior at 37°C, which is the temperature required for in-use physical stability testing.

Preferably, the pH of the present formulations is about pH 7.0 to about 8.0 and, most preferably 7.6 to 8.0. The formulations are preferably prepared under basic conditions by mixing the obesity protein and preservative at a pH greater than pH 7.0. Preferably, the pH is about 7.6 to 8.0, and most preferably about pH 7.8. Ideally, a preservative and water solution is mixed at pH 7.6 to 8.0. Added to this solution is obesity protein in water. The pH is adjusted, as necessary, to about pH 7.6 to 8.0. The solution is then held until the components are dissolved, approximately 20 to 40 minutes, preferably about 30 minutes. The base used to adjust the pH of the formulation may be one or more pharmaceutically acceptable bases such as sodium hydroxide and potassium hydroxide. The preferred base is sodium hydroxide.

The formulations prepared in accordance with the present invention may be used in a syringe, injector, pumps or any other device recognized in the art for parenteral administration.

Preferably, the obesity protein employed in the formulations of the present invention is human obesity protein of SEQ ID NO:1.

5                   10                  15
Val Pro Ile Gln Lys Val Gln Asp Asp Thr Lys Thr Leu Ile Lys Thr

            20                  25                  30
Ile Val Thr Arg Ile Asn Asp Ile Ser His Thr Gln Ser Val Ser Ser

        35                  40                  45
Lys Gln Lys Val Thr Gly Leu Asp Phe Ile Pro Gly Leu His Pro Ile

    50                  55                  60
Leu Thr Leu Ser Lys Met Asp Gln Thr Leu Ala Val Tyr Gln Gln Ile

65                  70                  75                  80
Leu Thr Ser Met Pro Ser Arg Asn Val Ile Gln Ile Ser Asn Asp Leu

                85                  90                  95
Glu Asn Leu Arg Asp Leu Leu His Val Leu Ala Phe Ser Lys Ser Cys

            100                 105                 110
His Leu Pro Trp Ala Ser Gly Leu Glu Thr Leu Asp Ser Leu Gly Gly

        115                 120                 125
Val Leu Glu Ala Ser Gly Tyr Ser Thr Glu Val Val Ala Leu Ser Arg

    130                 135                 140
Leu Gln Gly Ser Leu Gln Asp Met Leu Trp Gln Leu Asp Leu Ser Pro

145
Gly Cys                                             (SEQ ID NO:1)

The obesity protein employed in the formulations of the present invention can be prepared by any of a variety of recognized peptide synthesis techniques including classical (solution) methods, solid phase methods, semi synthetic methods, and more recent recombinant DNA methods. The preparation of the human obesity protein is known and disclosed, for example, in Halaas Jeffrey L. et al., Science 269 (1995). The preparation of other mammalian obesity proteins are described in U.S. application serial number 08/445,305, filed May 19, 1995 (EP0 743 321); U.S. patent application serial number 08/452,228, filed May 26, 1995 (EP0 744 408); and provisional application, 60,003935, filed September 19, 1995, (EP-A 96306721.0); all of which are herein incorporated by reference.

The obesity proteins described herein may also be produced either by recombinant DNA technology or well known chemical procedures, such as solution or solid-phase peptide synthesis, or semi-synthesis in solution beginning with protein fragments coupled through conventional solution methods. Recombinant methods are preferred if a high yield is desired. The basic steps in the recombinant production of protein include:
a) construction of a synthetic or semi-synthetic (or isolation from natural sources) DNA encoding the obesity protein,
b) integrating the coding sequence into an expression vector in a manner suitable for the expression of the protein either alone or as a fusion protein,
c) transforming an appropriate eukaryotic or prokaryotic host cell with the expression vector, and
d) recovering and purifying the recombinantly produced protein.
Synthetic genes, the in vitro or in vivo transcription and translation of which will result in the production of the protein may be constructed by techniques well known in the art. Owing to the natural degeneracy of the genetic code, the skilled artisan will recognize that a sizable yet definite number of DNA sequences may be constructed which encode the desired proteins. In the preferred practice of the invention, synthesis is achieved by recombinant DNA technology.

Methodology of synthetic gene construction is well known in the art. For example, see Brown, et al. (1979) Methods in Enzymology, Academic Press, N.Y., Vol. 68, pgs. 109-151. The DNA sequence corresponding to the synthetic claimed protein gene may be generated using conventional DNA synthesizing apparatus such as the Applied Biosystems Model 380A or 380B DNA synthesizers (commercially available from Applied Biosystems, Inc., 850 Lincoln Center Drive, Foster City, CA 94404). It may desirable in some applications to modify the coding sequence of the obesity protein so as to incorporate a convenient protease sensitive cleavage site, e.g., between the signal peptide and the structural protein facilitating the controlled excision of the signal peptide from the fusion protein construct.

The gene encoding the obesity protein may also be created by using polymerase chain reaction (PCR). The template can be a cDNA library (commercially available from CLONETECH or STRATAGENE) or mRNA isolated from the desired arrival adipose tissue. Such methodologies are well known in the art Maniatis, et al. Molecular Clonina: A Laboratory Manual, Cold Spring Harbor Press, Cold Spring Harbor Laboratory, Cold Spring Harbor, New York (1989).

The constructed or isolated DNA sequences are useful for expressing the obesity protein either by direct expression or as fusion protein. When the sequences are used in a fusion gene, the resulting product will require enzymatic or chemical cleavage. A variety of peptidases which cleave a polypeptide at specific sites or digest the peptides from the amino or carboxy termini (e.g. diaminopeptidase) of the peptide chain are known. Furthermore, particular chemicals (e.g. cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. See U.S. Patent No. 5,126,249; Carter P., Site Specific Proteolysis of Fusion Proteins, Ch. 13 in Protein Purification: From Molecular Mechanisms to Large Scale Processes, American Chemical Soc., Washington, D.C. (1990).

Construction of suitable vectors containing the desired coding and control sequences employ standard ligation techniques. Isolated plasmids or DNA fragments are cleaved, tailored, and religated in the form desired to form the plasmids required.

To effect the translation of the desired protein, one inserts the engineered synthetic DNA sequence in any of a plethora of appropriate recombinant DNA expression vectors through the use of appropriate restriction endonucleases. A synthetic coding sequence may be designed to possess restriction endonuclease cleavage sites at either end of the transcript to facilitate isolation from and integration into these expression and amplification and expression plasmids. The isolated cDNA coding sequence may be readily modified by the use of synthetic linkers to facilitate the incorporation of this sequence into the desired cloning vectors by techniques well known in the art. The particular endonucleases employed will be dictated by the restriction endonuclease cleavage pattern of the parent expression vector to be employed. The restriction sites are chosen so as to properly orient the coding sequence with control sequences to achieve proper in-frame reading and expression of the protein.

In general, plasmid vectors containing promoters and control sequences which are derived from species compatible with the host cell are used with these hosts. The vector ordinarily carries a replication origin as well as marker sequences which are capable of providing phenotypic selection in transformed cells. For example, E. coli is typically transformed using pBR322, a plasmid derived from an E. coli species (Bolivar, et al., Gene 2: 95 (1977)). Plasmid pBR322 contains genes for ampicillin and tetracycline resistance and thus provides easy means for identifying transformed cells. The pBR322 plasmid, or other microbial plasmid must also contain or be modified to contain promoters and other control elements commonly used in recombinant DNA technology.

The desired coding sequence is inserted into an expression vector in the proper orientation to be transcribed from a promoter and ribosome binding site, both of which should be functional in the host cell in which the protein is to be expressed. An example of such an expression vector is a plasmid described in Belagaje et al., U.S. patent No. 5,304,493, the teachings of which are herein incorporated by reference. The gene encoding A-C-B proinsulin described in U.S. patent No. 5,304,493 can be removed from the plasmid pRB182 with restriction enzymes NdeI and BamHI. The isolated DNA sequences can be inserted into the plasmid backbone on a NdeI/BamHI restriction fragment cassette.

In general, procaryotes are used for cloning of DNA sequences in constructing the vectors useful in the invention. For example, E. coli K12 strain 294 (ATCC No. 31446) is particularly useful. Other microbial strains which may be used include E. coli B and E. coli X1776 (ATCC No. 31537). These examples are illustrative rather than limiting.

Procaryotes also are used for expression. The aforementioned strains, as well as E. coli W3110 (prototrophic, ATCC No. 27325), bacilli such as Bacillus subtilis, and other enterobacteriaceae such as Salmonella typhimurium or Serratia marcescans, and various pseudomonas species may be used. Promoters suitable for use with prokaryotic hosts include the β-lactamase (vector pGX2907 [ATCC 39344] contains the replicon and β-lactamase gene) and lactose promoter systems (Chang et al., Nature, 275:615 (1978); and Goeddel et al., Nature 281:544 (1979)), alkaline phosphatase, the tryptophan (trp) promoter system (vector pATH1 [ATCC 37695] is designed to facilitate expression of an open reading frame as a trpE fusion protein under control of the trp promoter) and hybrid promoters such as the tac promoter (isolatable from plasmid pDR540 ATCC-37282). However, other functional bacterial promoters, whose nucleotide sequences are generally known, enable one of skill in the art to ligate them to DNA encoding the protein using linkers or adaptors to supply any required restriction sites. Promoters for use in bacterial systems also will contain a Shine-Dalgarno sequence operably linked to the DNA encoding protein.

The DNA molecules may also be recombinantly produced in eukaryotic expression systems. Preferred promoters controlling transcription in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. β-actin promoter. The early and late promoters of the SV40 virus are conveniently obtained as an SV40 restriction fragment which also contains the SV40 viral origin of replication. Fiers, et al., Nature, 273:113 (1978). The entire SV40 genome may be obtained from plasmid pBRSV, ATCC 45019. The immediate early promoter of the human cytomegalovirus may be obtained from plasmid pCMBb (ATCC 77177). Of course, promoters from the host cell or related species also are useful herein.

Transcription of the DNA by higher eucaryotes is increased by inserting an enhancer sequence into the vector. Enhancers are cis-acting elements of DNA, usually about 10-300 bp, that act on a promoter to increase its transcription. Enhancers are relatively oriented and positioned independently and have been found.5' (Laimins, L. et al., PNAS 78: 993 (1981)) and 3' (Lusky, M. L., et al., Mol. Cell Bio. 3:1108 (1983)) to the transcription unit, within an intron (Banerji, J. L. et al., Cell 33:729 (1983)) as well as within the coding sequence itself (Osborne, T. F., et al., Mol. Cell Bio. 4:1293 (1984)). Many enhancer sequences are now known from mammalian genes (globin, RSV, SV40, EMC, elastase, albumin, alpha-fetoprotein and insulin). Typically, however, one will use an enhancer from a eukaryotic cell virus. Examples include the SV40 late enhancer, the cytomegalovirus early promoter enhancer, the polyoma enhancer on the late side of the replication origin, and adenovirus enhancers.

Expression vectors used in eukaryotic host cells (yeast, fungi, insect, plant, animal, human or nucleated cells from other multicellular organisms) will also contain sequences necessary for the termination of transcription which may affect mRNA expression. These regions are transcribed as polyadenylated segments in the untranslated portion of the mRNA encoding protein. The 3' untranslated regions also include transcription termination sites.

Expression vectors may contain a selection gene, also termed a selectable marker. Examples of suitable selectable markers for mammalian cells are dihydrofolate reductase (DHFR, which may be derived from the BslII/HindIII restriction fragment of pJOD-10 [ATCC 68815]), thymidine kinase (herpes simplex virus thymidine kinase is contained on the BamHI fragment of vP-5 clone [ATCC 2028]) or neomycin (G418) resistance genes (obtainable from pNN414 yeast artificial chromosome vector [ATCC 37682]). When such selectable markers are successfully transferred into a mammalian host cell, the transfected mammalian host cell can survive if placed under selective pressure. There are two widely used distinct categories of selective regimes. The first category is based on a cell's metabolism and the use of a mutant cell line which lacks the ability to grow without a supplemented media. Two examples are: CHO DHFR⁻ cells (ATCC CRL-9096) and mouse LTK⁻ cells (L-M(TK-) ATCC CCL-2.3). These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in nonsupplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, Southern P. and Berg, P., J. Molec. Appl. Genet. 1: 327 (1982), mycophenolic acid, Mulligan, R. C. and Berg, P. Science 209:1422(1980), or hygromycin, Sugden, B. et al., Mol Cell. Biol. 5:410-413 (1985). The three examples given above employ bacterial genes under eukaryotic control to convey resistance to the appropriate drug G418 or neomycin (geneticin), xgpt (mycophenolic acid) or hygromycin, respectively.

A preferred vector for eucaryotic expression is pRc/CMV. pRc/CMV is commercially available from Invitrogen Corporation, 3985 Sorrento Valley Blvd., San Diego, CA 92121. To confirm correct sequences in plasmids constructed, the ligation mixtures are used to transform E. coli K12 strain DH10B (ATCC 31446) and successful transformants selected by antibiotic resistance where appropriate. Plasmids from the transformants are prepared, analyzed by restriction and/or sequence by the method of Messing, et al., Nucleic Acids Res. 9:309 (1981).

Host cells may be transformed with the expression vectors of this invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinarily skilled artisan. The techniques of transforming cells with the aforementioned vectors are well known in the art and may be found in such general references as Maniatis, et al., Molecular Cloning: A Laboratory Manual, Cold Spring Harbor Press, Cold Spring Harbor Laboratory, Cold Spring Harbor, New York (1989), or Current Protocols in Molecular Biology (1989) and supplements.

Preferred suitable host cells for expressing the vectors encoding the claimed proteins in higher eucaryotes include: African green monkey kidney line cell line transformed by SV40 (COS-7, ATCC CRL-1651); transformed human primary embryonal kidney cell line 293, (Graham, F. L. et al., J. Gen Virol. 36:59-72 (1977), Virology 77:319-329, Virology 86:10-21); baby hamster kidney cells (BHK-21(C-13), ATCC CCL-10, Virology 16:147 (1962)); Chinese hamster ovary cells CHO-DHFR⁻ (ATCC CRL-9096), mouse Sertoli cells (TM4, ATCC CRL-1715, Biol. Reprod. 23:243-250 (1980)); African green monkey kidney cells (VERO 76, ATCC CRL-1587); human cervical epitheloid carcinoma cells (HeLa, ATCC CCL-2); canine kidney cells (MDCK, ATCC CCL-34); buffalo rat liver cells (BRL 3A, ATCC CRL-1442); human diploid lung cells (WI-38, ATCC CCL-75); human hepatocellular carcinoma cells (Hep G2, ATCC HB-8065);and mouse mammary tumor cells (MMT 060562, ATCC CCL51).

In addition to prokaryotes, unicellular eukaryotes such as yeast cultures may also be used. Saccharomyces cerevisiae, or common baker's yeast is the most commonly used eukaryotic microorganism, although a number of other strains are commonly available. For expression in Saccharomyces, the plasmid YRp7, for example, (ATCC-40053, Stinchcomb, et al., Nature 282:39 (1979); Kingsman et al., Gene 7:141 (1979); Tschemper et al., Gene 10:157 (1980)) is commonly used. This plasmid already contains the trp gene which provides a selection marker for a mutant strain of yeast lacking the ability to grow in tryptophan, for example ATCC no. 44076 or PEP4-1 (Jones, Genetics 85:12 (1977)).

Suitable promoting sequences for use with yeast hosts include the promoters for 3-phosphoglycerate kinase (found on plasmid pAP12BD ATCC 53231 and described in U.S. Patent No. 4,935,350, June 19, 1990) or other glycolytic enzymes such as enolase (found on plasmid pAC1 ATCC 39532), glyceraldehyde-3-phosphate dehydrogenase (derived from plasmid pHcGAPC1 ATCC 57090, 57091), zymomonas mobilis (United States Patent No. 5,000,000 issued March 19, 1991), hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which contain inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein (contained on plasmid vector pCL28XhoLHBPV ATCC 39475, United States Patent No. 4,840,896), glyceraldehyde 3-phosphate dehydrogenase, and enzymes responsible for maltose and galactose (GAL1 found on plasmid pRyl21 ATCC 37658) utilization. Suitable vectors and promoters for use in yeast expression are further described in R. Hitzeman et al., European Patent Publication No. 73,657A. Yeast enhancers such as the UAS Gal from Saccharomyces cerevisiae (found in conjunction with the CYC1 promoter on plasmid YEpsec--hIlbeta ATCC 67024), also are advantageously used with yeast promoters.

The following examples will help describe how the invention is practiced and will illustrate the invention. The scope of the present invention is not to be construed as merely consisting of the following examples.

### Preparation 1

### Porcine OB Gene and Gene Product

Total RNA was isolated from porcine fat tissue obtained from Pel-Freez^{®}, Pel-Freez Inc., and the cDNA was cloned in accordance with the techniques described in Hsiung et al., Neuropeptide 25: 1-10 (1994).

Primers were designed based on the published amino acid sequence of the human *ob* gene. The primers were prepared for use in polymerase chain reaction (PCR) amplification methods using a Model 380A DNA synthesizers (PE-Applied Biosystems, Inc., 850 Lincoln Center Drive, Foster City, CA 94404). Primers PCROB-1 (12504) (ATG CAT TGG GGA MCC CTG TG), PCROB-2 (12505) (GG ATT CTT GTG GCT TTG GYC CTA TCT), PCROB-3 (12506) (TCA GCA CCC AGG GCT GAG GTC CA), and PCROB-4 (12507) (CAT GTC CTG CAG AGA CCC CTG CAG CCT GCT CA) were prepared.

For cDNA synthesis, total RNA 1 µL (1 µg/µl) isolated from porcine adipose tissue and 1 µL Perkin Elmer Random primers (50 µM) in a total volume of 12 µL were annealed for 10 minutes at 70°C and then cooled on ice. The following were then added to the annealed mixture: 4 µL of BRL 5x H-reverse transcriptase (RT) reaction buffer (Gibco-BRL CAT#28025-013), 2 µL of 0.1 M DTT, 1 µL of 10 mM dNTPs. This annealed mixture was then incubated at 37°C for 2 minutes before adding 1 µL BRL M-MLV-reverse transcriptase (200 U/µL) (CAT#28025-013) and incubated at 37°C for additional 1 hour. After incubation the mixture was heated at 95°C for 5 minutes and then was cooled on ice.

For amplification of cDNA, the polymerase chain reaction (PCR) was carried out in a reaction mixture (100 µL) containing the above cDNA reaction mixture (1 µL), 2.5 units of AmpliTaq DNA polymerase (Perkin-Elmer Corporation) 10 µl of 10x PCR reaction buffer (Perkin-Elmer Corporation) and 50 pmol each of the sense (PCROB-1) and antisense (PCROB-3) primers for porcine OB amplification. The condition for PCR was 95°C for 1 minute, 57°C for 1 minute and 72⁻°C for 1 minute for 30 cycles using a PCR DNA Thermal Cycler (Perkin-Elmer Corporation). After PCR amplification, 5 µL BRL T4 DNA polymerase (5 U/µL), 2 µL BRL T4 polynucleotide kinase (10 U/µL), and 5 µL ATP (10 nM) were added to the PCR reaction mixture (100 µl) directly and incubated for 30 minutes at 37°C. After the incubation the reaction mixture was heated at 95°C for 5 minutes and then was cooled on ice. The 500 bp fragment (∼0.5 mg) was purified by agarose gel electrophoresis and isolated by the freeze-squeeze method. The 500 bp fragment (∼0.2 µg) was then ligated into SmaI linearized pUC18 plasmid (∼1 µg) and the ligation mixture was used to transform DH5α (BRL) competent cells. The transformation mixture was plated on 0.02% X-Gal TY broth plates containing ampicillin (Amp) (100 µg/mL) and was then incubated overnight at 37°C. White clones were picked and were grown at 37°C overnight in TY broth containing Amp (100 µg/mL). The plasmid was isolated using a Wizard Miniprep DNA purification system (Promega) and submitted for DNA seqeuncing on a Applied Biosystem 370 DNA sequencer.

### Preparation 2

### Bovine OB Gene and Gene Product

The DNA sequence of the bovine OB gene was obtained by techniques analogous to Example 1, except sense (PCROB-2) and antisense (PCROB-3) primers were used for bovine OB cDNA amplification.

### Preparation 3

### Vector Construction

A plasmid containing the DNA sequence encoding the obesity protein is constructed to include NdeI and BamHI restriction sites. The plasmid carrying the cloned PCR product is digested with NdeI and BamHI restriction enzymes. The small ∼ 450bp fragment is gel-purified and ligated into the vector pRB182 from which the coding sequence for A-C-B proinsulin is deleted. The ligation products are transformed into E. coli DH10B (commercially available from GIBCO-BRL) and colonies growing on tryptone-yeast (DIFCO) plates supplemented with 10 mg/mL of tetracycline are analyzed. Plasmid DNA is isolated, digested with NdeI and BamHI and the resulting fragments are separated by agarose gel electrophoresis. Plasmids containing the expected ∼ 450bp NdeI to BamHI fragment are kept. E. coli K12 RV308 (available from the NRRL under deposit number B-15624) are transformed with this second plasmid, resulting in a culture suitable for expressing the protein.

The techniques of transforming cells with the aforementioned vectors are well known in the art and may be found in such general references as Maniatis, et al. (1988) Molecular Cloning: A Laboratory Manual, Cold Spring Harbor Press, Cold Spring Harbor Laboratory, Cold Spring Harbor, New York or Current Protocols in Molecular Biology (1989) and supplements. The techniques involved in the transformation of E. coli cells used in the preferred practice of the invention as exemplified herein are well known in the art. The precise conditions under which the transformed E. coli cells are cultured is dependent on the nature of the E. coli host cell line and the expression or cloning vectors employed. For example, vectors which incorporate thermoinducible promoter-operator regions, such as the c1857 thermoinducible lambda-phage promoter-operator region, require a temperature shift from about 30 to about 40 degrees C. in the culture conditions so as to induce protein synthesis.

In the preferred embodiment of the invention, E. coli K12 RV308 cells are employed as host cells but numerous other cell lines are available such as, but not limited to, E. coli K12 L201, L687, L693, L507, L640, L641, L695, L814 (E. coli B). The transformed host cells are then plated on appropriate media under the selective pressure of the antibiotic corresponding to the resistance gene present on the expression plasmid. The cultures are then incubated for a time and temperature appropriate to the host cell line employed.

Proteins that are expressed in high-level bacterial expression systems characteristically aggregate in granules or inclusion bodies which contain high levels of the overexpressed protein. Kreuger et al., in Protein Folding, Gierasch and King, eds., pgs 136-142 (1990), American Association for the Advancement of Science Publication No. 89-18S, Washington, D.C. Such protein aggregates must be solubilized to provide further purification and isolation of the desired protein product. Id. A variety of techniques using strongly denaturing solutions such as guanidinium-HCl and/or weakly denaturing solutions such as dithiothreitol (DTT) are used to solubilize the proteins. Gradual removal of the denaturing agents (often by dialysis) in a solution allows the denatured protein to assume its native conformation. The particular conditions for denaturation and folding are determined by the particular protein expression system and/or the protein in question.

Preferably, the DNA sequences are expressed with a dipeptide leader sequence encoding Met-Arg or Met-Tyr as described in U.S. Patent No. 5,126,249, herein incorporated by reference. This approach facilitates the efficient expression of proteins and enables rapid conversion to the active protein form with Cathepsin C or other dipeptidylpeptidases. The purification of proteins is by techniques known in the art and includes reverse phase chromatography, affinity chromatography, and size exclusion.

The following examples and preparations are provided merely to further illustrate the preparation of the formuations of the invention. The scope of the invention is not construed as merely consisting of the following examples.

### Example 1

### Human Obesity Protein Formulation

To generate a solution of Human Obesity Protein (hOb), the lyophilized solid material was first dissolved in water to generate a stock solution (stock 1). The concentration of hOb in stock 1 was verified by UV/Vis Spectrophometry using the known extinction coefficient for hOb at the maximum spectral absorbance (279nm or 280nm), measuring the maximum spectral absorbance (279nm or 280nm), and utilizing the dilution factor. A stock preservative solution containing methylparaben was prepared by dissolving the solid in water (stock 2). A solution of hOb at 1.6 mg/mL was prepared by addition of an aliquot of stock 1 to a container which held an aliquot of stock 2 along with the required quantity of water, at an alkaline pH (7.8±0.3); adjusted if necessary with HCl or NaOH. After an appropriate incubation period at room temperature (30 minutes), the solution pH was examined and adjusted if necessary with trace µL quantities of HCl or NaOH, to yield an hOb solution at 1.6 mg/mL with 0.17% methylparaben pH 7.8±0.1. The solution was then hand-filtered using a glass syringe with an attached 0.22 µm syringe filter into a glass vial.

### Example 2

### Human Obesity Protein Solution

To generate a solution of Human Obesity Protein (hOb), the lyophilized solid material was first dissolved in water to generate a stock solution (stock 1). The concentration of hOb in stock 1 was verified by UV/Vis Spectrophometry using the known extinction coefficient for hOb at the maximum spectral absorbance (279nm or 280nm), measuring the maximum spectral absorbance (279nm or 280nm), and utilizing the dilution factor. A stock preservative solution containing chlorobutanol, for example, was prepared by dissolving the solid in water (stock 2). A solution of hOb at 1.6 mg/mL was prepared by addition of an aliquot of stock 1 to a container which held an aliquot of stock 2 along with the required quantity of water, at an alkaline pH (7.8±0.3); adjusted if necessary with HCl or NaOH. After an appropriate incubation period at room temperature (30 minutes), the solution pH was examined and adjusted if necessary with trace µL quantities of HCl or NaOH, to yield an hOb solution at 1.6 mg/mL with 0.50% chlorobutanol pH 7.8±0.1. The solution was then hand-filtered using a glass syringe with an attached 0.22 µm syringe filter into a glass vial.

### Example 3

### Human Obesity Protein Solution

To generate a solution of Human Obesity Protein (hOb), the solid bulk material, lyophilized from a neutral water solution, was redissolved in water to generate a stock solution (stock 1). The concentration of hOb in stock 1 was verified by Uv/Vis Spectrophometry by multiplying the minimum spectral absorbance (279nm or 280nm) by the dilution factor divided by the known extinction coefficient for hOb. A stock preservative solution containing methylparaben was prepared by dissolving the solid in water (stock 2). A stock of an isotonicity agent, such as glycerin was prepared by dissolving the neat liquid in water (stock 3). A stock of a physiologically-tolerated buffer, such as sodium phosphate was prepared by dissolving the solid in water (stock 4). A solution of hOb at 1.6 mg/mL was prepared by addition of an aliquot of stock 1 to a container which held an aliquot of stock 2 along with an aliquot of stock 3 along with the required quantity of water, adjusted if necessary with HCl or NaOH to an alkaline pH (7.8±0.3). After an appropriate incubation period at room temperature (30 minutes) an aliquot of stock 4 was added. The solution pH was then readjusted if necessary with trace µL quantities of HCl or NaOH, to yield an hOb solution at 1.6 mg/mL with 0.17% methylparaben, 16 mg/mL glycerin, and 14mM sodium phosphate at pH 7.8±0.1. The solution was then hand-filtered using a glass syringe with an attached 0.22µm syringe filter into a glass vial.

### Example 4

### Human Obesity Protein Solution

To generate a solution of Human Obesity Protein (hOb), the solid bulk material, lyophilized from a neutral water solution, was redissolved in water to generate a stock solution (stock 1). The concentration of hOb in stock 1 was verified by W/Vis Spectrophometry by multiplying the maximum spectral absorbance (279nm or 280nm) by the dilution factor divided by the known extinction coefficient for hOb. A stock preservative solution containing chlorobutanol was prepared by dissolving the solid in water (stock 2). A stock of an isotonicity agent, such as glycerin, was prepared by dissolving the neat liquid in water (stock 3). A stock of a physiologically-tolerated buffer, such as sodium phosphate was prepared by dissolving the solid in water (stock 4). A solution of hOb at 1.6 mg/mL was prepared by addition of an aliquot of stock 1 to a container which held an aliquot of stock 2 along with an aliquot of stock 3 along with the required quantity of water, adjusted if necessary with HCl or NaOH to an alkaline pH (7.8±0.3). After an appropriate incubation period at room temperature (30 minutes) an aliquot of stock 4 was added. The solution pH was then readjusted if necessary with trace µL quantities of HCl or NaOH, to yield an hOb solution,.for example, at 1.6 mg/mL with 0.5% chlorobutanol, 16 mg/mL glycerin, and 14mM sodium phosphate (if required by the formulation) at pH 7.8±0.1. The solution was then hand-filtered using a glass syringe with an attached 0.22µm syringe filter into a glass vial.

### Example 5

### Preparation of Human Obesity Protein Solution

To generate a preserved solution of human obesity protein (hOb), the individual solution components were added in succession to a neutral water solution, allowing each component to dissolve prior to addition of the next component. To the container were added: approximately 5-6 mL of sterile water (final solution volume = 10.00 mL) followed by preservative solids (1.51 mg of butylparaben; final concentration = 0.015%). The solution was mixed at medium speed with a Teflon stir bar and heat was applied to promote dissolution of the solids. Care was exercised to dissolve the preservative solids, but not to overheat or boil the solution. After dissolution was achieved (approximately 1-1.5 hr.), the solution was allowed to cool to room temperature while maintaining stirring. Glycerin (160.0 mg) was then added to the solution and stirred until dissolution was achieved. The final glycerin concentration was targeted to be 16 mg/mL. A 10.0 mg quantity of solid ethylenediamine tetracetate (EDTA) was added to the solution and stirred to dissolve (final concentration = 0.10%). Dibasic sodium phosphate crystals (17.0 mg) were then added and allowed to dissolve by stirring. The final phosphate concentration was targeted at 6.3 mM. Sucrose (600.0 mg) was then added and allowed to stir until dissolved (final concentration of 60 mg/mL). Bulk lyophilized hOb (115.4 mg) was added incrementally to the stirring solution, allowing each aliquot to dissolve prior to the addition of successive portions. The quantity of hOb added to the solution yielded a final concentration of 10.2 mg/mL. The amount added was determined by using the "as is" weight %, determined for the specific hOb lot used by UV/Vis spectrophotometry. Following the addition of all bulk ingredients, the pH of the solution was adjusted to 8.5 using approximately 11 µL of 10% NaOH to promote dissolution of the solids. After stirring the solution for a 10-15 minute period, the pH was decreased to 7.8 using approximately 4-5 µL of 10% HCl and the solution was allowed to stir for an additional 10 minutes. The solution was then transferred to a 10 mL volumetric flask, and brought to final volume with sterile water. The flask was then inverted 20x to thoroughly mix the contents and transferred to a glass sample vial where a recheck of the pH yielded 7.78. A trace quantity of 10% NaOH (approximately 1 µL) was added to yield a final pH of 7.83. The solution was then hand-filtered, using a 5 mL glass syringe with an attached 0.2µm syringe filter into a 20 mL glass vial, capped and stored refrigerated until needed.

The principles, preferred embodiments and modes of operation of the present invention have been described in the foregoing specification. The invention which is intended to be protected herein, however, is not to be construed as limited to the particular forms disclosed, since they are to be regarded as illustrative rather than restrictive. Variations and changes may be made by those skilled in the art without departing from the spirit of the invention.

## Claims

1. A soluble parenteral formulation, comprising an obesity protein and a preservative selected from the group consisting of alkylparaben, chlorobutanol, or a mixture thereof.

2. A formulation of Claim 1, wherein the preservative is methylparaben, ethylparaben, propylparaben, or butylparaben.

3. A formulation of Claim 2, wherein the concentration of protein is about 1.0 mg/mL to about 10 mg/mL.

4. A formulation of Claim 3, which further comprises an isotonicity agent.

5. A formulation of Claim 4, which further comprises a physiologically acceptable buffer.

6. A formulation of Claim 5, wherein the preservative is methylparaben, and the isotonicity agent is glycerin.

7. A formulation of any one of Claims 1 through 6, wherein the protein is the human obesity protein optionally having a Met- leader sequence.

8. A formulation of any one of Claims 1 through 6, wherein the protein is the human obesity protein.

9. A process for preparing a soluble parenteral formulation of any one of Claims 1 through 8, which comprises mixing an obesity protein and a preservative selected from the group consisting of alkylparaben, chlorobutanol, or a mixture thereof.

10. A formulation as claimed in any one of Claims 1 through 8 for use in the treatment of obesity.


SEQUENCE LISTING

(1) GENERAL INFORMATION:

(i) APPLICANT: ELI LILLY AND COMPANY
(B) STREET: Lilly Corporate Center
(C) CITY: Indianapolis
(D) STATE: Indiana
(E) COUNTRY: United States of America
(F) ZIP: 46285

(ii) TITLE OF INVENTION: Obesity Protein Formulation

(iii) NUMBER OF SEQUENCES: 5

(iv) CORRESPONDENCE ADDRESS:
(A) ADDRESSEE: C. M. Hudson
(B) STREET: Erl Wood Manor
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:
(A) APPLICATION NUMBER: 97300299.1
(B) FILING DATE: 17th January 1997

(2) INFORMATION FOR SEQ ID NO:1:

(i) SEQUENCE CHARACTERISTICS:
(A) LENGTH: 146 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:1:

Val Pro Ile Gln Lys Val Gln Asp Asp Thr Lys Thr Leu Ile Lys Thr
1               5                   10                  15

Ile Val Thr Arg Ile Asn Asp Ile Ser His Thr Gln Ser Val Ser Ser
            20                  25                  30

Lys Gln Lys Val Thr Gly Leu Asp Phe Ile Pro Gly Leu His Pro Ile
        35                  40                  45

Leu Thr Leu Ser Lys Met Asp Gln Thr Leu Ala Val Tyr Gln Gln Ile
    50                  55                  60

Leu Thr Ser Met Pro Ser Arg Asn Val Ile Gln Ile Ser Asn Asp Leu
65                  70                  75                  80

Glu Asn Leu Arg Asp Leu Leu His Val Leu Ala Phe Ser Lys Ser Cys
                85                  90                  95

His Leu Pro Trp Ala Ser Gly Leu Glu Thr Leu Asp Ser Leu Gly Gly
            100                 105                 110

Val Leu Glu Ala Ser Gly Tyr Ser Thr Glu Val Val Ala Leu Ser Arg
        115                 120                 125

Leu Gln Gly Ser Leu Gln Asp Met Leu Trp Gln Leu Asp Leu Ser Pro
    130                 135                 140

Gly Cys
145

(2) INFORMATION FOR SEQ ID NO:2:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:2:

ATGCATTGGG GACCCTGTG 19

(2) INFORMATION FOR SEQ ID NO:3:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:3:

GGATTCTTGT GGCTTTGGCC TATCT 25

(2) INFORMATION FOR SEQ ID NO:4:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:4:

TCAGCACCCA GGGCTGAGGT CCA 23

(2) INFORMATION FOR SEQ ID NO:5:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:5:

CATGTCCTGC AGAGACCCCT GCAGCCTGCT CA 32